(19) Europäisches Patentamt
European Patent Office
Office européen des brevets

(11) Veröffentlichungsnummer: **0 340 422**
**A2**

(12) # EUROPÄISCHE PATENTANMELDUNG

(21) Anmeldenummer: 89104285.5

(51) Int. Cl.⁴: **A61B 17/22**

(22) Anmeldetag: 10.03.89

(30) Priorität: 30.04.88 DE 3814743

(43) Veröffentlichungstag der Anmeldung:
08.11.89 Patentblatt 89/45

(84) Benannte Vertragsstaaten:
BE CH DE FR GB LI

(71) Anmelder: **Richard Wolf GmbH**
**Pforzheimer Strasse 32**
**D-7134 Knittlingen(DE)**

(72) Erfinder: **Wurster, Helmut, Dipl.-Ing.**
**Mozartstrasse 20**
**D-7519 Oberderdingen(DE)**

(74) Vertreter: **Wilcken, Thomas, Dipl.-Ing. et al**
**Musterbahn 1**
**D-2400 Lübeck(DE)**

(54) **Einrichtung zur Auflösung von Konkrementen in einer Körperhöhle.**

(57) Einrichtung zur Auflösung von Konkrementen (36) in einer Körperhöhle, beispielsweise in der Gallenblase (32), unter Zuhilfenahme eines in die die aufzulösenden Konkremente enthaltende Körperhöhle applizierten Lösungsmittels, wobei zur Bildung einer Dispersion ein extrakorporal an den Patienten ankoppelbarer und auf ausgewählte Bereiche der Körperhöhle fokussierbarer Ultraschallwandler (1) Ultraschallwellen in einem Frequenzbereich zwischen 20 und 40 kHz mit einer Energiedichte aussendet, die ausreicht, um in der Körperhöhlenflüssigkeit und/oder in dem Lösungsmittel Kavitationen zu erzeugen.

Fig.1

## Einrichtung zur Auflösung von Konkrementen in einer Körperhöhle

Es ist mittlerweile üblich geworden, in der klinischen Behandlung zur Beseitigung von Konkrementen in einer Körperhöhle, beispielsweise von Steinen in der Niere, diese unter Anwendung fokussierter Ultraschall-Stoßwellen zu zerstören. Nach der Applikation von Ultraschallwellen der genannten Art auf das Konkrement befinden sich Konkrementpartikel in der betreffenden Körperhöhle, welche über natürliche Ausgänge ausgeschwemmt oder mittels einer Saug-und Spülpumpe abgeführt werden.

Es hat sich nun im Laufe der Jahre der klinischen Anwendung sogenannter Lithotriptoren gezeigt, daß ihr Einsatz u.a. aufgrund anatomischer Gegebenheiten nicht auf allen Gebieten zum Erfolg führt.

Als Beispiel für ein klinisches Einsatzgebiet, bei dem der Einsatz von Ultraschall-Lithotriptoren nur bedingt möglich ist, sei hier die Zerstörung von Steinen in der Gallenblase genannt. Die eingeschränkte Anwendbarkeit ist hier darauf zurückzuführen, daß die durch die Steinzertrümmerung entstehenden Steinpartikel den Ductus cysticus nicht oder nicht vollständig passieren können.

Eine andere in Abhängigkeit der Steinart angewandte Methode zur Entfernung von Konkrementen aus Körperhöhlen ist die Applikation eines Lösungsmittels. Die Zuführung des Lösungsmittels erfolgt bei Anwendung dieser Methode zumeist perkutan. Bei dieser Methode wird es jedoch als Nachteil empfunden, daß die Steinauflösung durch das Lösungsmittel ein relativ langwieriger Prozeß ist und daß das perkutane Zuführen des Lösungsmittels und das notwendige perkutane Abführen des Gallensaftes zu einer Belastung des Patienten führt, die als nicht mehr tolerierbar angesehen werden muß. Die Langwierigkeit des Auflösungsprozesses ist unter anderem auch dadurch bedingt, daß die in Frage kommenden Lösungsmittel leichter als die Gallenflüssigkeit sind, so daß sich die Lösungsmittel aufgrund der sich während der Behandlung bildenden Gallenflüssigkeit in zunehmendem Maße auf dieser absetzen und damit deren Wirksamkeit herabsetzen.

Vor dem aufgezeigten Hintergrund des Standes der Technik ist es nun die Aufgabe der vorliegenden Erfindung, eine Einrichtung zu schaffen, mit deren Hilfe eine Vermischung zwischen der Gallenflüssigkeit und dem Lösungsmittel erzielbar ist, so daß ein Einwirken des Lösungsmittels auf die aufzulösenden Konkremente ermöglicht und die für die Auflösung benötigte Zeit in erheblichem Maße herabgesetzt wird.

Die Aufgabe der Erfindung wird gelöst durch eine Einrichtung mit den Merkmalen des Anspruches 1.

Weitere vorteilhafte Ausgestaltungen ergeben sich aus den Unteransprüchen.

Durch die von der erfindungsgemäßen Einrichtung bewirkten Kavitationen in der Körperflüssigkeit und/oder in dem Lösungsmittel werden diese miteinander dispergiert, um die Oberfläche des zu entfernenden Konkrementes ständig mit dem Lösungsmittel zu benetzen, wodurch die Einwirkung des Lösungsmittels auf das Konkrement erheblich intensiviert wird. Dadurch werden die eingangs genannten Nachteile der eingangs genannten Methode zur Auflösung von Konkrementen, welche aus den unterschiedlichen Dichten der Körperhöhlenflüssigkeit und der Lösungsmittel resultieren, beseitigt.

Die erfindungsgemäße Einrichtung besteht im wesentlichen aus einem extrakorporal an dem Patienten ankoppelbaren, fokussierbaren Ultraschallwandler, der Ultraschallwellen in einem Frequenzbereich zwischen 20 und 40 kHz mit einer Energiedichte aussendet, die ausreicht, die erwähnten Kavitationen zu erzeugen. Andererseits reicht die Energiedichte jedoch nicht aus, um das aufzulösende Konkrement, ähnlich wie bei der Anwendung eines Ultraschall-Lithotriptors, zu zertrümmern.

Vorteilhafterweise ist die Abschlußfläche des stirnseitig an die Körperoberfläche anzukoppelnden Endes des in Sandwich-Bauweise aufgebauten Ultraschallwandlers zur Fokussierung der Ultraschallwellen auf ausgewählte Bereiche der Körperhöhle konkav ausgebildet, wodurch in der Dispersion eine hohe Energiedichte erreicht wird.

Gemäß einer anderen vorteilhaften Ausgestaltung ist der Ultraschallwandler ein sphärischer Ultraschall-Resonator.

Um den Grad der Auflösung des in der Dispersion befindlichen Konkrementes während der Behandlung kontrollieren zu können, kann dem Ultraschallwandler vorteilhafterweise ein Ortungssystem zugeordnet sein. Dieses Ortungssystem kann beispielsweise aus einem B-Scanner bestehen. Aber auch eine röntgentechnische Kontrolle des Auflösungsgrades mittels einer derartigen Einrichtung ist möglich.

Die Erfindung wird anhand der Zeichnungen näher erläutert. Hierbei zeigt:

Figur 1 einen Ultraschallwandler in Sandwich-Bauweise, wie er bei einer Ausführung der erfindungsgemäßen Einrichtung zum Einsatz kommt,

Figur 2 eine weitere Ausführungsform des Ultraschallwandlers der Einrichtung als sphärischer Resonator, und

Figur 3 eine schematische Darstellung des Einsatzes der Einrichtung zur Auflösung von Konkrementen in der Gallenblase.

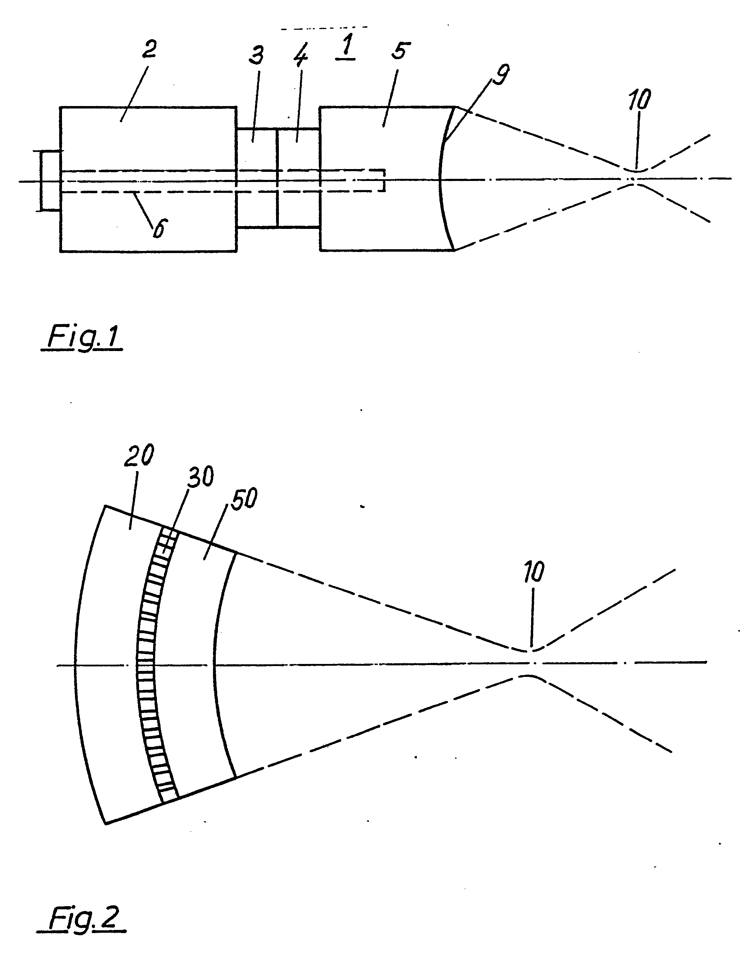

Figur 1 zeigt einen Ultraschallwandler 1, wie er bei einer Ausführungsform der erfindungsgemäßen Einrichtung zum Einsatz kommt. Hier ist der Ultraschallwandler 1 in Sandwich-Bauweise dargestellt, bestehend aus einem Stahlkörper 2, piezoelektrischen Keramikscheiben 3,4 und einem Aluminiumkörper 5, die alle über einen Bolzen 6 miteinander verspannt sind. Die piezoelektrischen Keramikscheiben werden elektrisch über Anschlüsse mit einem Generator 35 verbunden, so daß die vom Generator 35 erzeugten elektrischen Schwingungen in mechanische Schwingungen der Keramikscheiben umgesetzt werden.

Der Aluminiumkörper 5 des Wandlers 1 weist stirnseitig eine konkav ausgebildete Abschlußfläche 9 auf, welche an die Körperoberfläche anzukoppeln ist.

Durch die konkave Abschlußfläche 9 werden vom Wandler 1 erzeugte Ultraschallwellen in einem Fokus 10 fokussiert.

Figur 2 zeigt die Ausbildung eines Ultraschallwandlers als sphärischen Resonator, wie er in einer anderen Ausführungsform der erfindungsgemäßen Einrichtung verwendet werden kann. Dieser sphärische Resonator besteht aus einem sphärischen Stahlkörper 20, einer sphärischen Keramikschicht 30 aus piezoelektrischen Keramikkörpern sowie aus einem sphärischen Aluminiumkörper 50. Der über Anschlußkontakte mit einem Generator 35 elektrisch in Verbindung stehende Resonator strahlt Ultraschallwellen ab, die in einem Fokus 10 gebündelt sind.

Die erfindungsgemäße Einrichtung wird nun so betrieben, daß die Ultraschallwandler gemäß Figur 1 oder Figur 2 vom Generator 35 so angesteuert werden, daß die Wandler Ultraschallwellen in einem Frequenzbereich von 20 bis 40 kHz und mit einer solchen Energie abstrahlen, die ausreicht, um in der Körperhöhlenflüssigkeit und/oder in dem Lösungsmittel Kavitationen zu erzeugen. Hierbei ist der Fokus des Ultraschallwandlers koinzident mit dem Innenraum der Körperhöhle, in welcher sich das aufzulösende Konkrement befindet.

Nach dem Zuführen von Lösungsmittel für das Konkrement in die Körperhöhle wird die Einrichtung in dem oben erwähnten Sinne betrieben, so daß das Lösungsmittel in der Körperhöhle mit der Körperhöhlenflüssigkeit dispergiert. Dadurch wird erreicht, daß die Einwirkung des Lösungsmittels auf das zu entfernende Konkrement erheblich intensiviert wird.

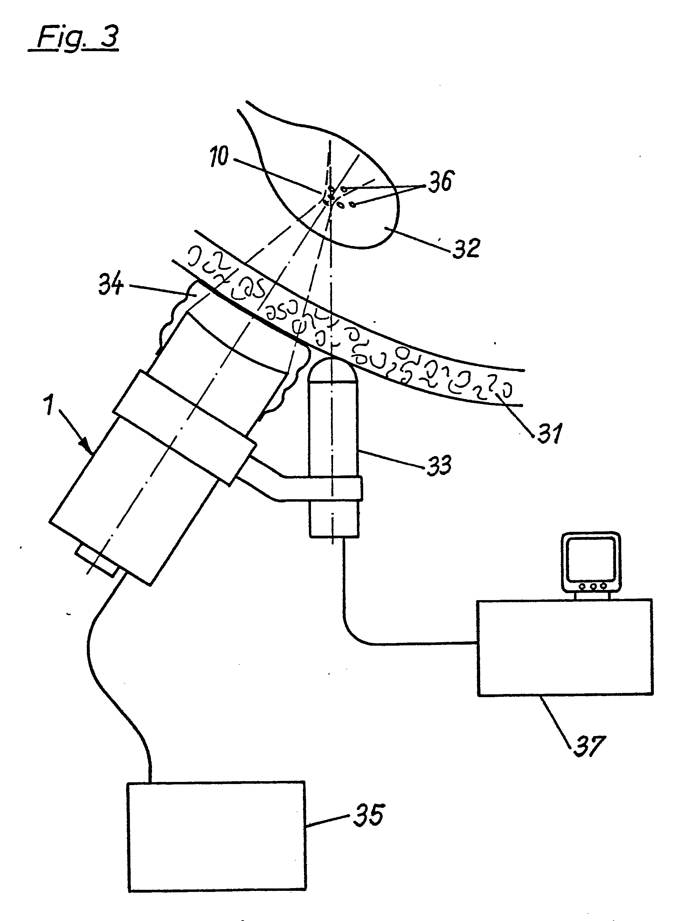

Figur 3 zeigt eine schematische Darstellung der Einrichtung beim Einsatz während einer Therapie zur Auflösung eines Konkrementes 36 in der Gallenblase 32. Hierbei sind die vom Wandler 1 herrührenden Ultraschallwellen wieder in einem Fokus 10 fokussiert, der koinzident mit dem Inneren der Gallenblase 32 ist. Im übrigen erfolgt die Ankopplung des Wandlers 1 an die Körperoberfläche 31 in an sich bekannter Weise, bspw. über einen mit Wasser gefüllten flexiblen Sack 34. Auch in diesem Ausführungsbeispiel wird der Wandler 1 durch einen Generator 35 angesteuert.

Dem Wandler 1 der Einrichtung gemäß Figur 3 ist ein Ortungssystem 33 zugeordnet, das im dargestellten Fall als B-Scanner ausgebildet ist, dessen Signale in an sich bekannter Weise auf einem Monitor 37 darstellbar sind. Zur räumlichen Abbildung des oder der Körpersteine können auch mehrere Ortungssysteme zur Anwendung kommen.

Mit Hilfe des Ortungssystems 33 ist es zusätzlich zur Steinortung und Positionierung der Konkremente im Fokus des Wandlers möglich, den Auflösungsgrad des Konkrementes 36 während der Therapie zu kontrollieren, so daß erforderlichenfalls zur Beibehaltung der Wirkung des Lösungsmittels ein zyklischer oder kontinuierlicher Austausch des Lösungsmittels erfolgen kann.

In Abweichung von der Ausführungsform gemäß Figur 3 ist es selbstverständlich möglich, die Beobachtung röntgentechnisch durchzuführen mit einer derartigen (nicht dargestellten) Einrichtung.

## Ansprüche

1. Einrichtung zur Auflösung von Konkrementen in einer Körperhöhle, beispielsweise in der Gallenblase, unter Zuhilfenahme eines in die aufzulösende Konkremente enthaltende Körperhöhle applizierten Lösungsmittels, gekennzeichnet durch einen extrakorporal an den Patienten ankoppelbaren und auf den Innenraum der Körperhöhle fokussierbaren Ultraschallwandler (1), der Ultraschallwellen in einem Frequenzbereich von 20 bis 40 kHz und mit einer solchen Energie aussendet, die ausreicht, um in der Körperhöhlenflüssigkeit und/oder in dem Lösungsmittel Kavitationen zu erzeugen, um das Lösungsmittel mit der Körperhöhlenflüssigkeit zu dispergieren.

2. Einrichtung nach Anspruch 1, dadurch gekennzeichnet, daß der Ultraschallwandler (1) in Sandwich-Bauweise aufgebaut ist, wobei sein stirnseitiges, an die Körperoberfläche anzukoppelndes Wandlerende eine konkav ausgebildete Abschlußfläche (9) aufweist.

3. Einrichtung nach Anspruch 1, dadurch gekennzeichnet, daß der Ultraschallwandler ein sphärischer Ultraschall-Resonator ist.

4. Einrichtung nach einem der Ansprüche 1 bis 3, dadurch gekennzeichnet, daß dem Ultraschallwandler ein Ortungssystem (33) zugeordnet ist, mit Hilfe dessen die Position von Körperhöhlensteinen ermittelt, der Fokus des Ultraschall wandlers auf die Körperhöhlensteine ausgerichtet und der Grad der Auflösung der sich in der Dispersion befindlichen Konkremente (36) kontrolliert werden kann.

_Fig.1_

_Fig.2_

_Fig. 3_